# EUROPÄISCHE PATENTANMELDUNG

(11) **EP 2 388 284 A1**
(43) Veröffentlichungstag der Anmeldung: **23.11.2011**
(21) Anmeldenummer: 10163621.5
(22) Anmeldetag: 21.05.2010
(51) Int. Cl.: C08G 18/28, C08G 18/36, C08G 18/42, C08G 18/79, C07C 267/00, C08K 5/00, C08K 5/29

(54) **Biobasierte Carbodiimide, ein Verfahren zu deren Herstellung und deren Verwendung**

(71) Anmelder: Rhein Chemie Rheinau GmbH, 68219 Mannheim (DE)
(72) Erfinder: Laufer, Wilhelm, 67158 Ellerstadt (DE); Eiben, Robert, 68623 Lampertheim (DE); Eckert, Armin, 68794 Oberhausen (DE); Orsini, Franco, 67294 Ilbesheim (DE)
(74) Vertreter: Siegers, Britta

(57) **Zusammenfassung**

Die Erfindung betrifft neue biobasierte Carbodiimide, ein Verfahren zu deren Herstellung und deren Verwendung. Bei den erfindungsgemäßen biobasierten Carbodiimiden handelt es sich um das Umsetzungsprodukt von mit mindestens einem Carbodiimid und einer aus nachwachsenden Rohstoffen isolierten oder daraus hergestellten H-aciden Verbindung mit einer Funktionalität >1 und/oder einem Hydroxycarbonsäureester mit 2-24-Kohlenstoffatomen.

## Beschreibung

Die Erfindung betrifft neue biobasierte Carbodiimide, ein Verfahren zu deren Herstellung und deren Verwendung. Bei den erfindungsgemäßen biobasierten Carbodiimide handelt es sich um das Umsetzungsprodukt von mindestens einem Carbodiimid und einer aus nachwachsenden Rohstoffen isolierten oder daraus hergestellten H-aciden Verbindung mit einer Funktionalität >1 und/oder einem Hydroxycarbonsäureester mit 2-24-Kohlenstoffatomen.

Organische Carbodiimide sind bekannt und finden beispielsweise Verwendung als Stabilisator gegen den hydrolytischen Abbau von Estergruppen enthaltenden Verbindungen. Hauptanwendungsgebiet sind z.B. Polyadditions- und Polykondensationsprodukte, vor allem Polyurethane und thermoplastische Polyester.

Bei Polymeren wird es zunehmend wichtiger, auf natürliche Rohstoffe, wie z.B. biobasierende Polyole (natural oil polyols), zurückzugreifen. So hat beispielsweise die US-Regierung 2002 ein neues Programm ins Leben gerufen, nach dem solche Produkte als "Grünes" Produkt deklariert werden dürfen, wenn diese in dem hier beabsichtigten Anwendungsbereich der Polyurethane zur Hausisolierung zu mehr als 7% eines biobasierenden Rohstoffes aufweisen.

Diese "Grünen Produkte" haben mittlerweile auch Einzug gefunden bei der Herstellung von Polyurethanen und Schäumen, siehe US- A- 7125950, US-A- 7566406.

Es besteht daher ein Bedarf, diese "Grünen Produkte" auch in dem Hydrolysestabilisatorbereich einsetzbar zu machen.

Aufgabe der vorliegenden Erfindung war es daher, neue Carbodiimide bereitzustellen, die als "Grünes" Produkt einstufbar sind und gleich gute Eigenschaften aufweisen, wie die nach dem Stand der Technik bekannten Carbodiimide.

Die dieser Erfindung zugrunde liegenden Aufgabe konnte durch die neuen modifizierten Carbodiimide gelöst werden, die biobasiert sind.

Gegenstand der vorliegenden Erfindung sind daher biobasierte Carbodiimide, erhältlich aus der Umsetzung von mindestens einem Carbodiimid und mindestens einer aus nachwachsenden Rohstoffen isolierten oder daraus hergestellten H-aciden Verbindung mit einer Funktionalität >1 und/oder einem Hydroxycarbonsäureester.

Im Rahmen der vorliegenden Erfindung geeignete Carbodiimide sind insbesondere monomere oder polymere Carbodiimide.

Carbodiimide im Sinne der Erfindung sind vorzugsweise Verbindungen der allgemeinen Formel

R'-(-N=C=N-R-)ₘ-R" (I),

in der
R einen aromatischen, aliphatischen, cycloaliphatischen oder araliphatischen Rest bedeutet, der in dem Fall eines aromatischen oder eines araliphatischen Restes in mindestens einer ortho-Stellung, bevorzugt in beiden ortho-Stellungen zum aromatischen Kohlenstoffatom, das die Carbodiimidgruppe trägt, aliphatische und/oder cycloaliphatische Substituenten mit mindestens 2 Kohlenstoffatomen, bevorzugt verzweigte oder cyclische aliphatische Reste mit mindestens 3 Kohlenstoffatomen, insbesondere Isopropylgruppen, tragen kann,
R' = C₁ - C₁₈-Alkyl, C₅-C₁₈-Cycloalkyl-, Aryl, C₇-C₁₈- Aralkyl oder R-NCO, R-NHCONHR¹, R-NHCONR¹R² oder R-NHCOOR³ und
R" -NCO bedeutet,
wobei in R' unabhängig voneinander R¹ und R² gleich oder verschieden sind und einen C₁ - C₆-Alkyl-, C₆-C₁₀-Cycloalkyl- oder C₇-C₁₈-Aralkylrest darstellen und R³ eine der Bedeutungen von R¹ hat oder einen Polyester- oder einen Polyamidrest bedeutet, wie z.B. Acetat- oder Butyl-und/oder Benzoeester und
m einer ganze Zahl von 1 bis 5.000, bevorzugt von 1 bis 500, entspricht.

Bei den Resten R handelt es sich bei den aromatischen Resten vorzugsweise um einen Arylrest. Bei den araliphatischen Resten handelt es sich vorzugsweise um einen C₇-C₁₈- Aralkylrest, wobei im Aralkylrest die "N=C=N"-Gruppe sowohl über den Alkylrest als auch über den Arylrest gebunden sein kann,
bei den aliphatischen Resten um linear oder verzweigtes, gegebenenfalls substituiertes C₁ - C₁₈-Alkyl und
bei den cycloaliphatischen Resten um gegebenenfalls substituiertes C₅-C₁₉-Cyclo- oder Bicycloalkyl.

Besonders geeignet sind Carbodiimide der allgemeinen Formel (II), die in den ortho-Stellungen zur Carbodiimidgruppe durch Isopropyl substituiert sind und die in der para-Stellung zur Carbodiimidgruppe ebenfalls durch Isopropyl substituiert sind, wie z.B. 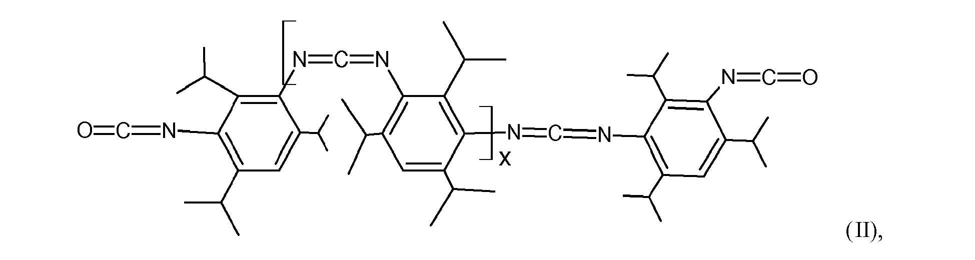 mit x = 1 bis 50, bevorzugt 2 -20.

In einer weiteren bevorzugten Ausführungsform der Erfindung handelt es sich bei dem Carbodiimid um eine Verbindung der Formel (III) 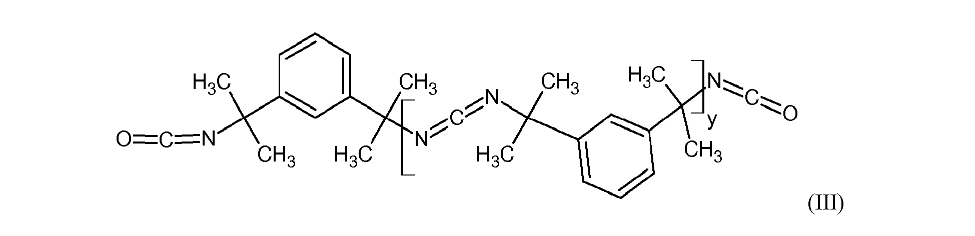 mit y = 1 bis 20, bevorzugt 2 - 8.

Ferner können auch polymere aliphatische Carbodiimide verwendet werden, beispielsweise auf Basis von Isophorondiisocyanat oder Dicyclohexylmethan-4,4'-di-isocyanat (H12-MDI = hydriertes MDI).

Bei den vorgenannten Carbodiimiden handelt es sich um käuflich erhältliche Verbindungen, die z.B. bei der Rhein Chemie Rheinau GmbH unter den Handelsnamen Stabaxol® P100 (N-C-N-Gehalt: 13-14%), Stabaxol® P400, (N-C-N-Gehalt: 13-14%) und Stabaxol® P 220 (N-C-N-Gehalt: 13-14 %) käuflich erhältlich sind. Auch die von der Firma Raschig vertriebenen Produkte mit der Bezeichnung Stabilisator 9000 und 11000 können im Rahmen der vorliegenden Erfindung verwendet werden.

Ebenso möglich ist auch die Herstellung der Carbodiimide nach den beispielsweise in US 2,941,956 beschrieben Verfahren oder durch die Kondensation von Diisocyanaten unter Abspaltung von Kohlendioxid bei erhöhten Temperaturen, z.B. bei 40 °C bis 200 °C, in Gegenwart von Katalysatoren. Geeignete Verfahren werden in DE-A-11 30 594 und in der FR 1 180 370 beschrieben. Als Katalysatoren haben sich z.B. starke Basen oder Phosphorverbindungen bewährt. Vorzugsweise werden Phospholenoxide, Phospholidine oder Phospholinoxide sowie die entsprechenden Sulfide verwendet. Ferner können als Katalysatoren tertiäre Amine, basisch reagierende Metallverbindungen, Carbonsäuremetallsalze und nicht basische Organometallverbindungen verwendet werden.

Zur Herstellung der eingesetzten Carbodiimide und/oder Polycarbodiimide eignen sich alle Isocyanate, wobei im Rahmen der vorliegenden Erfindung bevorzugt Carbodiimide und/oder Polycarbodiimide verwendet werden, die auf durch C₁- bis C₄-Alkyl substituierten aromatischen Isocyanaten aufbauen, wie z.B. 2,6-Diisopropylphenylisocyanat, 2,4,6-Triisopropylphenyl-1,3-diisocyanat, 2,4,6-Triethylphenyl-1,3-diisocyanat, 2,4,6-Trimethylphenyl-1,3-diisocyanat, 2,4'-Diisocyanatodiphenylmethan, 3,3',5,5'-Tetraisopropyl-4,4'-diisocyanatodiphenylmethan, 3,3',5,5'-Tetraethyl-4,4'-diisocyanatodiphenylmethan, Tetramethylxyloldiisocyanat, 1,5-Naphthalindiisocyanat, 4,4'-Diphenylmethandiisocyanat, 4,4'-Diphenyldimethylmethandiisocyanat, 1,3-Phenylendiisocyanat, 1,4-Phenylendiisocyanat, 2,4-Tolylendiisocyanat, 2,6-Tolylendiisocyanat, ein Gemisch aus 2,4-Tolylendiisocyanat und 2,6-Tolylendiisocyanat, Hexamethylendiisocyanat, Cyclohexan-1,4-diisocyanat, Xylylendi-isocyanat, Isophorondiisocyanat, Dicyclohexylmethan-4,4'-di-isocyanat, Methylcyclohexandi-isocyanat, Tetramethylxylylendiisocyanat, 2,6-Diisopropylphenylenisocyanat und 1,3,5-Triisopropylbenzol-2,4-diisocyanat oder deren Gemische, oder auf substituierten Aralkylen, wie 1,3-Bis-(1-methyl-1-isocyanato-ethyl)-benzol, basieren. Besonders bevorzugt ist es, wenn die Carbodiimide und/oder Polycarbodiimide auf 2,4,6-Triisopropylphenyl-1,3-diisocyanat basieren. Polycarbodiimide können außerdem, wenn sie aus Isocyanaten hergestellt worden sind, noch reaktionsfähige NCO-Gruppen und komplex gebundene monomere Isocyanate enthalten.

In einer weiteren Ausführungsform der vorliegenden Erfindung ist es auch möglich, eine Mischung verschiedener Carbodiimide einzusetzen. Wenn eine Mischung von Carbodiimiden verwendet wird, so können die verwendeten Carbodiimide ausgewählt sein aus der Gruppe der monomeren polymeren Carbodiimide, wobei auf obige Ausführungen im Hinblick auf die Verbindungen der allgemeinen Formeln (I) bis (III) verwiesen wird.

Der Begriff Funktionalität im Sinne der Erfindung umfaßt nur die reaktiven Gruppen, die mit Isocyanaten zu substituierten Urethan- und/oder Harnstoffderivaten reagieren können.

Bei den Hydroxycarbonsäureestern im Sinne der Erfindung handelt es sich vorzugsweise um Hydroxycarbonsäureester mit 2-24-Kohlenstoffatomen, wie z.B. Polymilchsäure und/oder Polyhydroxybutyrate der Formel (IV) 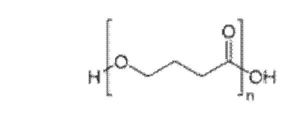 mit n = 2 -20.

Bei den aus nachwachsenden Rohstoffen isolierten H-aciden Verbindungen mit einer Funktionalität >1 handelt es sich vorzugsweise um natürliche Polyole, wie z.B. Rizinusöl, Stärke und/oder Zucker.

Bei den vorgenannten Verbindung handelt es sich um handelsübliche Substanzen.

Bei den aus nachwachsenden Rohstoffen hergestellten H-aciden Verbindungen mit einer Funktionalität >1 handelt es sich vorzugsweise um Glycerin, Polyole aus Pflanzenölen, wie z.B. Rapsöl, Sojaöl und/oder aus ungesättigten Fettsäuren, wie z.B. Ölsäure.

Bei den vorgenannten Verbindung handelt es sich um handelsübliche Substanzen, die z.B. bei der Firma Cargill und/oder Urethane Soy Systems erhältlich sind.

Die Umwandlung aus Pflanzenölen bzw. aus ungesättigten Fettsäuren kann nach den dem Fachmann geläufigen Verfahren, wie z.B. durch Ozonisierung mit nachfolgender Glykolyse, Epoxidierung mit anschließender Ringöffnung durch z.B. Alkohole oder Hydroformylierung und anschließende Reduktion mit Wasserstoff erfolgen.

In einer weiteren Ausführungsform der vorliegenden Erfindung werden die aus nachwachsenden Rohstoffen isolierten oder daraus hergestellten H-aciden Verbindungen mit einer Funktionalität >1 und/oder die Hydroxycarbonsäureester im Gemisch mit den gängigen nicht biobasierten Polyolen auf Polyether-, Polyester- und/oder Polyetheresterbasis, erhältlich bei den Firmen Bayer AG, BASF AG etc., eingesetzt.

Der Anteil an biobasierten Rohstoffen sollten vorzugsweise 7%, bezogen auf das biobasierte Carbodiimid, nicht unterschreiten.

Die erfindungsgemäßen biobasierten Carbodiimide lassen sich dabei vorzugsweise über die Reaktion mindestens eines Carbodiimids mit mindestens einer aus nachwachsenden Rohstoffen isolierten oder daraus hergestellten H-aciden Verbindung mit einer Funktionalität >1 und/oder dem Hydroxycarbonsäureester bei Temperaturen zwischen 20 und 200°C, vorzugsweise 80 - 90°C, in Masse und/oder in Lösung in Gegenwart oder in Abwesenheit mindestens eines Katalysator herstellen.

Gegenstand der vorliegenden Erfindung ist zudem ein Verfahren zur Herstellung der erfindungsgemäßen Carbodiimide, wonach mindestens ein Carbodiimid mit mindestens einer aus nachwachsenden Rohstoffen isolierten oder daraus hergestellten H-aciden Verbindung mit einer Funktionalität >1 und/oder dem Hydroxycarbonsäureester und gegebenenfalls nicht biobasierten Polyolen auf Polyether-, Polyester- und/oder Polyetheresterbasis bei Temperaturen zwischen 20 und 200°C, vorzugsweise 80 - 90°C, in Masse und/oder in Lösung gegebenenfalls in Gegenwart eines Katalysators umgesetzt werden.

Für die Umsetzung in Lösung werden vorzugsweise als Lösungsmittel Toluol, Xylol, Essigester, Essigsäurebutylester und/oder Methylethylketon eingesetzt, wobei für die Umsetzung je nach Lösungsmittel Temperaturen von 80 -120°C bevorzugt sind.

Als Katalysatoren kommen alle für diese Umsetzung bekannten Katalysatoren in Frage. Bevorzugt hierfür sind: tert. Amine, in der Matrix lösliche Zinn-Verbindungen, wie z.B. DibutylZinndilaurat, Titan-Verbindungen, wie z.B. Alkalititanate und/ oder aber Blei, Bismut und/oder Zinkverbindungen.

Die so erhaltenen modifizierten (biobasierten) Carbodiimide erfüllen das Kriterium eines "Grünen" Produktes.

Das Verhältnis des Carbodiimid zu der H-aciden Verbindung bzw. dem Hydroxycarbonsäureester beträgt vorzugsweise 5:95 bis 95:5, bevorzugt 7: 93 bis 60:40, besonders bevorzugt 7: 93 bis 50:50.

Die Umsetzung der H-acide Verbindung bzw. den Hydroxycarbonsäureester mit dem Carbodiimid erfolgt dabei vorzugsweise bei Temperaturen von 20 °C bis 200 °C, bevorzugt 25 °C bis 150 °C, besonders bevorzugt 80 °C bis 120 °C, wobei die Mischzeit von 0,1 min bis 360 min, bevorzugt 1 min bis 180 min, besonders bevorzugt 5 min bis 120 min betragen kann.

Die erfindungsgemäßen Carbodiimide eignen sich hervorragend als Akzeptor für freie Carbonsäuren und finden daher vorzugsweise Verwendung als Stabilisatoren gegen den hydrolytischen Abbau von Estergruppen enthaltenden Verbindungen, beispielsweise Estergruppen enthaltende Polymere, z.B. Polykondensationsprodukte, wie beispielsweise thermoplastische Polyester, wie Polyethylen- und -butylenterephthalat, Polyetherester, Polyamide, Polyesteramide, Polycaprolactone sowie ungesättigte Polyesterharze und Polyesterester, wie z.B. Blockcopolymere aus Polyethylen- oder Butylenterephthalat und Polycaprolacton und Polyadditionsprodukte, z.B. Polyurethane, Polyharnstoffe und Polyurethan-Polyhamstoff-Elastomere, die Estergruppen enthalten.

Diese Estergruppen enthaltenden Verbindungen sind allgemein bekannt. Ihre Ausgangssubstanzen, Herstellverfahren, Strukturen und Eigenschaften sind in der Standardliteratur vielfältig beschrieben. Aufgrund der guten Löslichkeit in den Aufbaukomponenten zur Herstellung von Polyurethanen und der guten Verträglichkeit mit den gebildeten Polyurethanen eignen sich die erfindungsgemäßen (Poly)carbodiimide insbesondere als Stabilisatoren gegen den hydrolytischen Abbau von Polyurethanen, vorzugsweise kompakten oder zelligen Polyurethan-Elastomeren und insbesondere thermoplastischen Polyurethanen sowie zelligen oder kompakten Elastomeren.

Gegenstand der vorliegenden Erfindung ist daher zudem die Verwendung der erfindungsgemäßen Carbodiimide in Polyurethan-Anwendungen, für Hartschaum/Coatings, Weichschaum, CASE (Coatings Adhesives Sealants Elastomers), Thermoplasten, für den Hydrolyseschutz, in Schmierstoffanwendungen, wie z.B. esterbasierenden Ölen, in Transformatorenölen und für die Vernetzung von Polyurethanen.

Die nachfolgenden Beispiele dienen der Erläuterung der Erfindung, ohne dabei limitierend zu wirken.

### Ausführungsbeispiele

### Beispiel 1: Herstellung des biobasierten Carbodiimids A (erfindungsgemäß)

101,0g Stabaxol® P220, ein polymeres Carbodiimid auf Basis von Tetramethyl-xylylendiisocyanat (NCN-Gehalt ca. 14%), wurde unter Stickstoff vorgelegt und auf 100 °C erwärmt.

Zugegeben wurde 41,0 g Polyethylenglykol-(Monomethylether) mit der OHZ (OH-Zahl) = 165,7 und 38,5 g Soyol® R2-052-C (biobasierendes Polyesterpolyol der Firma Urethane Soy Systems = Biopolyol) mit der OH-Zahl 65,2. Anschließend wurde noch 330 min gerührt, über Nacht abgestellt und am nächsten Tag auf 120°C erwärmt und noch 240 min bei 120°C gerührt. Der Anteil an Biopolyol beträgt 21 %.

### Säureabbau mit diesem biobasierten Carbodiimid A

Wie bekannt, kann die Wirkung eines Hydrolyseschutzmittels auf Basis sterisch gehinderter Carbodiimide in flüssigen Polyesterpolyolen mittels Säureabbau geprüft werden.

Die Wirkung des biobasierten Carbodiimids wurde in dem biobasierenden Polyesterpolyol (Soyol® R2-052-C der Firma Urethane Soy Systems = Biopolyol) geprüft.

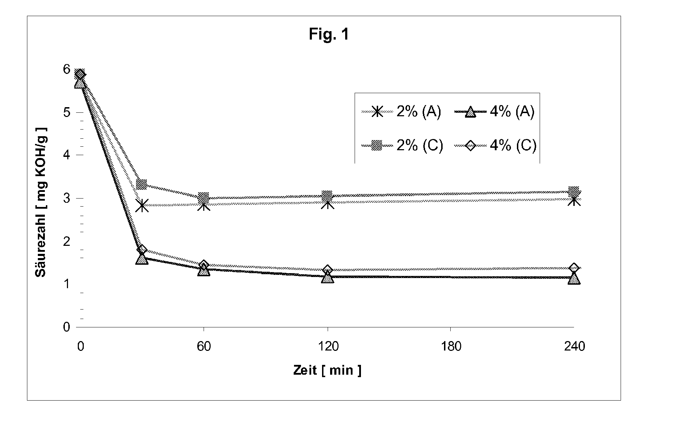

Bei 100°C wurden 2% bzw. 4% oben genannten biobasierten Carbodiimids A (Proben (A)) in das Biopolyol eingerührt und regelinäßig die Säurezahl gemessen. Die Ergebnisse sind in Figur 1 dargestellt.

In beiden Fällen ist der Säureabbau klar zu erkennen. Die Wirkung des biobasierten Carbodiimids ist damit vergleichbar mit dem nicht-biobasierten Carbodiimid Stabaxol^{®} P 200 (Proben (C)), siehe Figur 1, die zum Vergleich auch 1 und 2% von (C) (Stabaxol® P 200) enthält.

### Beispiel 2: Herstellung des biobasierten Carbodiimids B (erfindungsgemäß)

200g Stabaxol® P 220, ein polymeres Carbodiimid auf Basis von Tetramethyl-xylylendiisocyanat (NCN-Gehalt ca. 14%), wurde unter Stickstoff vorgelegt und auf 140 °C erwärmt.

Zugegeben wurden 238 g Agrol 2.0, ein biobasiertes Polyol der Firma Cargill mit der OH-Zahl 74,5.

Anschließend wurde so lange gerührt, bis der NCO-Gehalt auf Null abgesunken ist. Der Anteil an Biopolyol betrug 54 %.

### Säureabbau mit diesem biobasierten Carbodiimid B

Die Wirkung des biobasierten Carbodiimids B wurde in dem biobasierenden Polyesterpolyol (Soyol® T 22-60-C der Firma Urethane Soy Systems = Biopolyol) geprüft.

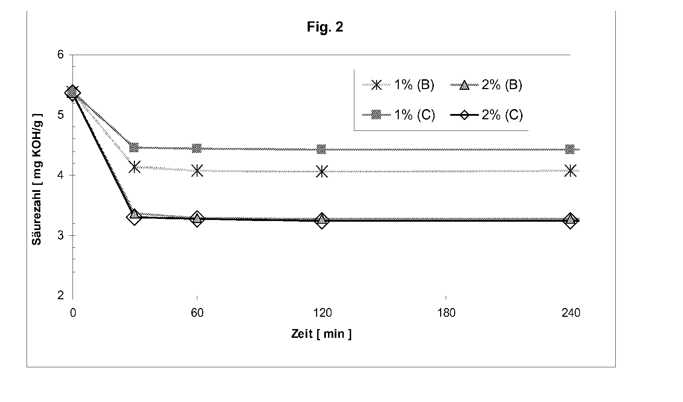

Bei 100°C wurden 1% bzw. 2% oben genannten biobasierten Carbodiimids B (Proben (B)) in das Biopolyol eingerührt und regelinäßig die Säurezahl gemessen. Die Ergebnisse sind in Figur 2 dargestellt. In beiden Fällen ist der Säureabbau klar zu erkennen.

Die Wirkung des biobasierten Carbodiimids ist damit vergleichbar mit nicht biobasierten Carbodiimid Stabaxol® P 200 (Proben (C)), siehe. Figur 2, die zum Vergleich auch 1 und 2% von (C) (Stabaxol® P 200) enthält.

## Patentansprüche

1. Biobasierte Carbodiimide, erhältlich aus der Umsetzung von mindestens einem Carbodiimid und einer aus nachwachsenden Rohstoffen isolierten oder daraus hergestellten H-aciden Verbindung mit einer Funktionalität >1 und/oder einem Hydroxycarbonsäureester mit 2-24-Kohlenstoffatomen.

2. Biobasierte Carbodiimide nach Anspruch 1, **dadurch gekennzeichnet, dass** es sich bei dem Carbodiimid um eine Verbindung der Formel (I)
R'-(-N=C=N-R-)ₘ-R" (I)
handelt, in der
R für einen aromatischen, aliphatischen, cycloaliphatischen oder araliphatischen Rest steht, der in dem Fall eines aromatischen oder eines araliphatischen Restes in mindestens einer ortho-Stellung zum aromatischen Kohlenstoffatom, das die Carbodiimidgruppe trägt, aliphatische und/oder cycloaliphatische Substituenten mit mindestens 2 Kohlenstoffatomen, tragen kann,
R' = C₁ - C₁₈-Alkyl, C₅-C₁₈-Cycloalkyl-, Aryl, C₇-C₁₈- Aralkyl oder R-NCO, R-NHCONHR¹, R-NHCONR¹R² oder R-NHCOOR³ und
R"-NCO bedeutet,
wobei in R' unabhängig voneinander R¹ und R² gleich oder verschieden sind und einen C₁ - C₆-Alkyl-, C₆-C₁₀-Cycloalkyl- oder C₇-C₁₈-Aralkylrest darstellen und R³ eine der Bedeutungen von R¹ hat oder einen Polyester- oder einen Polyamidrest bedeutet
und
m einer ganze Zahl von 1 bis 5.000, bevorzugt von 1 bis 500, entspricht.

3. Biobasierte Carbodiimide nach Anspruch 1 oder 2, **dadurch gekennzeichnet, dass** es sich bei dem Carbodiimid um eine Verbindung der Formeln (II)- (III) 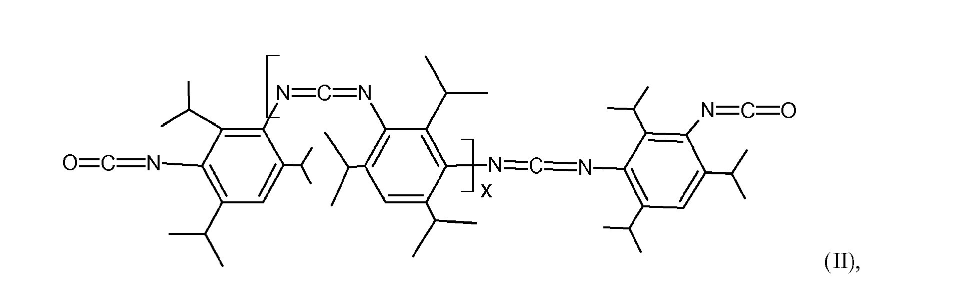 mit x = 1 bis 50, 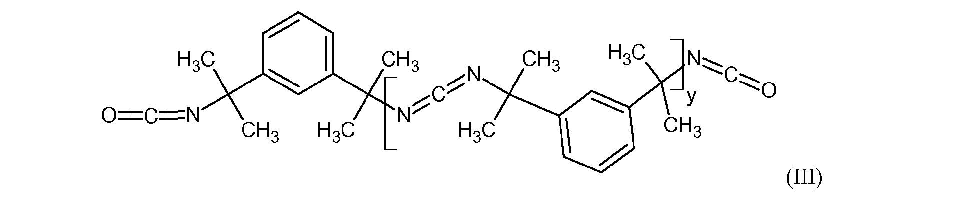 mit y = 1 bis 20
und/oder Isophorondiisocyanat und/oder Dicyclohexylmethan-4,4'-di-isocyanat (H12 MDI (hydriertes MDI).

4. Biobasierte Carbodiimide nach einem der Ansprüche 1 bis 3, **dadurch gekennzeichnet, dass** es sich bei der H-aciden Verbindung mit einer Funktionalität >1 und/oder dem Hydroxycarbonsäureester um eine oder mehrere der nachstehenden Verbindungen handelt:
Hydroxycarbonsäureester mit 2-24-Kohlenstoffatomen, Polyhydroxybutyrate der Formel (IV) 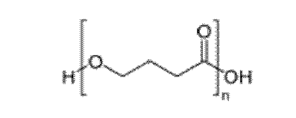
mit n = 2 -20,
natürliche Polyole, Glycerin, Polyole aus Pflanzenölen und/oder aus ungesättigten Fettsäuren.

5. Biobasierte Carbodiimide nach einem der Ansprüche 1 bis 4, **dadurch gekennzeichnet, dass** das Verhältnis des Carbodiimid zu der H-aciden Verbindung und/oder dem Hydroxycarbonsäureester 5:95 bis 95:5 beträgt.

6. Biobasierte Carbodiimide nach einem der Ansprüche 1 bis 5, **dadurch gekennzeichnet, dass** diese zusätzlich nicht biobasierte Polyole auf Polyether-, Polyester- und/oder Polyetheresterbasis enthalten.

7. Verfahren zur Herstellung der biobasierten Carbodiimide nach einem der Ansprüche 1 bis 6, **dadurch gekennzeichnet, dass** mindestens ein Carbodiimid mit mindestens einer aus nachwachsenden Rohstoffen isolierten oder daraus hergestellten H-aciden Verbindung mit einer Funktionalität >1 und/oder dem Hydroxycarbonsäureester und gegebenenfalls nicht biobasierten Polyolen auf Polyether-, Polyester- und/oder Polyetheresterbasis bei Temperaturen zwischen 20 und 200°C in Masse und/oder in Lösung gegebenenfalls in Gegenwart eines Katalysators umgesetzt werden.

8. Verwendung der biobasierten Carbodiimide nach einem der Ansprüche 1 bis 6 in Polyurethan-Anwendungen, für Hartschaum/Coatings, Weichschaum, CASE (Coatings Adhesives Sealants, Elastomers), Thermoplasten, in Schmierstoffanwendungen, in Transformatorenölen für den Hydrolyseschutz und für die Vernetzung von Polyurethanen.
